# EUROPÄISCHE PATENTSCHRIFT

(11) **EP 2 904 962 B1**
(45) Veröffentlichungstag und Bekanntmachung des Hinweises auf die Patenterteilung: **07.08.2019**
(21) Anmeldenummer: 15151807.3
(22) Anmeldetag: 20.01.2015
(51) Int. Cl.: A61B 1/12, A61B 1/00, G02B 23/24, G02B 27/00, A61B 90/70

(54) **Kamerasystem**
Camera system
Système de caméra

(30) Priorität: 05.02.2014 DE 102014202075
(43) Veröffentlichungstag der Anmeldung: 12.08.2015
(73) Patentinhaber: Deutsches Zentrum für Luft- und Raumfahrt e.V., 51147 Köln (DE)
(72) Erfinder: Bahls, Thomas, 86947 Weil (DE); Fröhlich, Florian Alexander, 82110 Germering (DE)
(74) Vertreter: dompatent von Kreisler Selting Werner - Partnerschaft von Patent- und Rechtsanwälten mbB

(56) Entgegenhaltungen:
- WO-A1-2014/048972
- JP-A- 2005 052 229
- US-A- 5 575 756
- US-A- 6 009 971
- US-A1- 2012 092 765
- US-B1- 6 254 386

## Beschreibung

Die Erfindung betrifft ein Kamerasystem, insbesondere für die minimalinvasive Chirurgie zur Reinigung einer Kamera für die minimalinvasive Chirurgie.

In der minimalinvasiven Chirurgie werden über Arbeitstrokare spezielle Instrumente sowie Optiken in den Patienten eingeführt. Zu diesem Zweck sind lediglich kleine Hautschnitte notwendig. Der Vorteil der minimalinvasiven Chirurgie liegt z. B. in kleineren Narben, schnellerer Genesung, geringerem Infektionsrisiko und Verkürzung des Krankenhausaufenthalts.

Operationen in der minimalinvasiven Chirurgie gehen oft mit längeren Operationszeiten im Vergleich zur offenen Chirurgie einher. Aus diesem Grund ist es wichtig, die Anzahl der Instrumentenwechsel zu minimieren. Neben dem zeitlichen Aspekt wird der Chirurg durch die Instrumentenwechsel in seinem Arbeitsfluss immer wieder unterbrochen. Zu den eigentlichen Instrumentenwechseln müssen je nach Operation in regelmäßigen Abständen die Optiken gereinigt werden, da diese beschlagen oder von Körperflüssigkeiten bespritzt werden können. Hierzu muss die Optik aus dem Patientenkörper entnommen, gereinigt und danach wieder eingeführt werden. Dies ist nach Aussage eines Arztes ca. alle 10 Minuten notwendig. Bei langen Operationen kann dies so zu 20 bis 30 Reinigungszyklen führen.

Aufgabe der Erfindung ist es, ein Kamerasystem, insbesondere für die minimalinvasive Chirurgie zu schaffen, bei dem die Reinigung einer Kamera vereinfacht ist. Eine Vorrichtung gemäß dem Oberbegriff des unabhängigen Anspruchs 1 ist aus der Druckschrift US 2012/092765 A1 bekannt.

Die Aufgabe wird erfindungsgemäß gelöst durch ein Kamerasystem gemäß Anspruch 1.

Das erfindungsgemäße Kamerasystem weist eine Kamera mit einer Optik auf. Insbesondere ist die Kamera geeignet für die minimalinvasive Chirurgie, so dass vorzugsweise die Optik endoskopisch ausgebildet ist. Die Optik weist insbesondere mindestens eine Oberfläche auf, die bei herkömmlichen Kameras dem Einfluss der Umgebung ausgesetzt ist. Durch die Optik werden Bilder des Operationssitus auf eine Beobachtungsvorrichtung übertragen. Dabei ist die tatsächliche Ausgestaltung der Übertragung und der Beobachtungsvorrichtung für die vorliegende Erfindung nicht erheblich.

Erfindungsgemäß ist mit der Optik mindestens eine transparente Folie verbunden, wobei die Folie entfernbar ist. Insbesondere ist die Folie mit der mindestens einen Oberfläche verbunden. Bevorzugt ist mindestens eine Folie unmittelbar mit der mindestens einen Oberfläche verbunden. Schmutz, der sich auf der Optik absetzt und diese verdeckt, wodurch die Bildqualität reduziert wird, wird durch Entfernen der Folie ebenfalls von der Optik entfernt. Die Folie ist dabei transparent für sichtbares Licht, d.h. sie besitzt für sichtbares Licht eine geringe intrinsische Absorption. Alternativ oder zusätzlich hierzu kann die Folie aber auch transparent sein im Bereich des Infrarot.

Vorzugsweise überdeckt die Folie die mindestens eine Oberfläche der Optik vollständig.

Insbesondere kann es sich bei der transparenten Folie um eine Kunststofffolie handeln. Soll die transparente Folie intrakorporal von der Optik entfernt werden, muss die transparente Folie, falls es sich hierbei um eine Kunststofffolie handelt, aus dem Körper entweder durch Absaugen oder durch ein Herausführen vom Operationssitus entfernt und vorzugsweise aus dem Körper herausgeführt werden. Alternativ hierzu kann die transparente Folie auch hergestellt sein aus einem biokompatiblen Material wie z. B. Gelatine, so dass die transparente Folie nach dem Entfernen von der Optik im Körper zurückgelassen werden kann und vom Körper resorbiert wird.

Insbesondere ist die Folie rückstandslos von der Optik entfernbar. Dabei erfolgt das Entfernen insbesondere durch ein Abziehen der Folie. Damit die Folie rückstandslos von der Optik entfernbar ist, kann die Folie eine lösbare Haftschicht aufweisen, mit der die Folie mit der Optik und insbesondere mit der mindestens einen Oberfläche der Optik verbunden ist. Alternativ hierzu kann die Verbindung der Optik mit der Folie durch die Van-Der-Waals-Kraft bzw. durch Adhäsion erfolgen.

Vorzugsweise ist die Optik mit einer Vielzahl von aufeinander angeordneten Folien verbunden. Dabei sind insbesondere die Folien einzeln entfernbar. Insbesondere wird durch die Vielzahl von aufeinander angeordneten Folien die Optik und vorzugsweise die mindestens eine Oberfläche der Optik vollflächig überdeckt. Durch das Vorsehen einer Vielzahl von aufeinander angeordneten Folien ist es möglich, die Optik der Kamera mehrfach durch das sukzessive Entfernen einzelner transparenten Folien zu reinigen, wobei durch jedes Entfernen einer einzelnen Folie Schmutz, der die Optik verdeckt ebenfalls entfernt wird. Vorzugsweise wird zum Reinigen die letzte Folie entfernt, betrachtet ausgehend von der Optik der Kamera.

Erfindungsgemäss weist die Folie mehrere Abschnitte auf, wobei eine Oberseite eines Abschnitts verbunden ist mit einem unmittelbar vorhergehenden Abschnitt und eine Unterseite des Abschnitts verbunden ist mit einem unmittelbaren nachfolgenden Abschnitt. Ein erster Abschnitt ist dabei insbesondere mit der Optik verbunden. Die einzelnen Abschnitte sind so aufeinandergefaltet, dass diese mäanderförmig angeordnet sind. Insbesondere wird die Optik gereinigt durch das Entfernen des jeweils letzten Abschnitts von dem unmittelbar vorhergehenden Abschnitt. Somit ist ein mehrfaches Reinigen der Optik möglich in Abhängigkeit von der Anzahl der vorgesehenen Abschnitte.

Vorzugsweise ist die Verbindung der einzelnen Abschnitte lösbar. Hierzu kann zwischen den einzelnen Abschnitten eine Haftschicht angeordnet sein, welche dann selbstverständlich transparent sein muss oder die Verbindung der einzelnen Abschnitte kann durch Adhäsion erfolgen.

Vorzugsweise weist die mindestens eine Folie eine Lasche auf zur Vereinfachung der Entfernung. Die Lasche kann dabei einfacher gegriffen werden. Dies ist insbesondere in der minimalinvasiven Chirurgie von großem Vorteil, da hier das Entfernen der Folie intrakorporal durch ein endoskopisches Instrument erfolgt. Besonders bevorzugt ist es hierbei, die Laschen entlang des Umfangs der Folie versetzt anzuordnen. Hierdurch überdecken sich die einzelnen Laschen nicht, was ein Greifen einer einzelnen Lasche weiter vereinfacht.

Vorzugsweise ist eine Entfernungsvorrichtung zum Entfernen der transparenten Folie vorgesehen. Bei der Entfernungsvorrichtung handelt es sich insbesondere um einen Greifer, der als separates ebenfalls endoskopisches Instrument ausgebildet sein kann.

Besonders bevorzugt ist es jedoch, dass die Entfernungsvorrichtung mit der Kamera verbunden ist. Hierdurch ist nur ein Instrument erforderlich. Insbesondere ist die Entfernungsvorrichtung ausgebildet, um bei Vorsehen einer Folie mit mehreren Abschnitten durch Ziehen an einer vorzugsweise vorgesehenen Lasche sukzessive die einzelnen Abschnitte der Folie von einander zu lösen.

Nachfolgend wird die Erfindung anhand der Zeichnungen näher erläutert.

Es zeigen:
- Figur 1: eine schematische Darstellung des erfindungsgemäßen Kamerasystems,
- Figur 2: eine Detailansicht der Optik eines Kamerasystems mit einer Vielzahl von Folien in Explosionsdarstellung und
- Figur 3: eine Detailansicht einer Optik des erfindungsgemäßen Kamerasystems mit einer mäanderförmig angeordneten Folie.

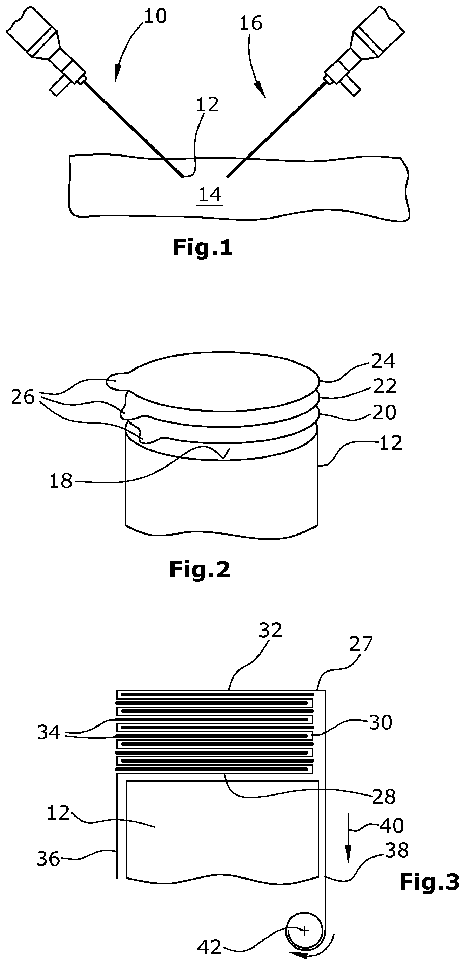

Das erfindungsgemäße Kamerasystem weist eine Kamera 10 mit einer Optik 12 auf, wobei mindestens eine Oberfläche der Optik 12 Einflüssen der Umgebung ausgesetzt ist, hierdurch insbesondere verschmutzt werden kann. Die Kamera 10 ist dabei ausgebildet als endoskopische Kamera für die minimalinvasive Chirurgie. In Figur 1 ist die Kamera 10 zur Beobachtung des Operationssitus 14 vorgesehen. Gemäß dem erfindungsgemäßen Verfahren kann eine Reinigungsvorrichtung 16, die ebenfalls als minimalinvasiv chirurgisches Instrument ausgebildet ist, an die Optik 12 herangeführt werden. Eventuell vorhandener Schmutz auf der Optik 12 kann nachfolgend durch die Reinigungsvorrichtung abgespült, abgeblasen oder abgestreift werden. Dieses Verfahren bietet sich insbesondere an, falls die Kamera 10 als auch die Reinigungsvorrichtung 16 robotergestützt insbesondere durch einen Operationsroboter geführt werden. In diesem Fall ist die Position der Optik 12 der Kamera 10 hinreichend bekannt, so dass die Reinigungsvorrichtung 16 präzise an die Optik 12 herangeführt werden kann.

In Figur 2 ist eine Detailansicht einer Optik 12 dargestellt als Explosionsdarstellung. Die Optik 12 weist eine Oberfläche 18 auf, die vollständig überdeckt ist durch mindestens eine transparente Folie 20. In Figur 2 sind drei transparente Folien 20, 22, 24 dargestellt. Die Folien 20, 22, 24 sind dabei aufeinander angeordnet und überdecken die Oberfläche 18 der Optik 12 vollständig. Weiter weisen die Folien 20, 22, 24 Laschen 26 auf. Die Laschen 26 sind dabei versetzt entlang des Umfangs der Folien angeordnet.

Hierdurch wird ein Greifen der Laschen vereinfacht und entsprechend wird ebenfalls das Entfernen der entsprechenden Folie vereinfacht.

Die erste Folie 20 ist unmittelbar mit der Oberfläche 18 der Optik 12 verbunden. Zur Reinigung der Optik 12 wird die jeweils letzte Folie 24 durch Abziehen entfernt. Der Schmutz auf der Folie 24 wird mit der Folie von der Optik entfernt.

Eine Folie 27 (Figur 3), die mit der Optik 12 verbunden ist, kann mehrere Abschnitte 30 aufweisen, wobei die Abschnitte 30 mäanderförmig angeordnet sind. Dabei weist die Folie 27 einen ersten Abschnitt 28 auf, der insbesondere direkt mit der Optik 12 verbunden ist. Weiter weist die Folie 27 einen letzten Abschnitt 32 auf. Die einzelnen Abschnitte sind durch Haftschichten 34 miteinander verbunden. Weiter weist die Folie 27 eine Haltelasche 36 auf, durch die eine bessere Verbindung der Folie 27 mit der Optik 12 gewährleistet wird. Darüber hinaus weist die Folie 27 eine Abziehlasche 38 auf. Durch Ziehen an der Abziehlasche 38 in Richtung des Pfeils 40 wird der letzte Abschnitt 32 der Folie 27 vom vorhergehenden Abschnitt gelöst. Bei kontinuierlichem Ziehen an der Abziehlasche 38 entlang des Pfeils 40 werden sukzessive alle Abschnitte vom letzten Abschnitt 32 bis zum ersten Abschnitt 28 voneinander gelöst. Hierdurch ist es ermöglicht die Optik 12 mehrfach zu reinigen und Schmutz zu entfernen. Insbesondere werden die einzelnen Abschnitte gelöst durch eine Entfernungsvorrichtung 42, durch welche die Folie beispielsweise aufgerollt wird und durch das Aufrollen an der Abziehlasche 38 entlang des Pfeils 40 gezogen wird.

## Patentansprüche

1. Endoskopische Kamera für die minimalinvasive Chirurgie, mit
einer eine Optik (12) aufweisende Kamera (10)
wobei die Optik (12) mit einer transparenten Folie (26) verbunden ist und die Folie (26) entfernbar ist,
**dadurch gekennzeichnet, dass** die Folie (26) mehrere Abschnitte (30) aufweist, wobei eine Oberseite des Abschnitts (30) verbunden ist mit einem unmittelbar vorhergehenden Abschnitt und eine Unterseite des Abschnitts (30) verbunden ist mit einem unmittelbar nachfolgenden Abschnitt., wobei die einzelnen Abschnitte so aufeinandergefaltet sind, dass diese mäanderförmig angeordnet sind.

2. Endoskopische Kamera nach Anspruch 1, **dadurch gekennzeichnet, dass** die Verbindung der einzelnen Abschnitte (30) lösbar ist und insbesondere zwischen den einzelnen Abschnitten (30) eine Haftschicht (34) angeordnet ist.

3. Endoskopische Kamera nach einem der Ansprüche 1 bis 2, **dadurch gekennzeichnet, dass** mindestens eine Folie (20, 22, 24, 26) eine Lasche (26, 36, 38) aufweist.

4. Endoskopische Kamera nach einem der Ansprüche 1 bis 3, **gekennzeichnet durch** eine Entfernungsvorrichtung (42) zum Entfernen der transparenten Folie (20, 22, 24, 26).

5. Endoskopische Kamera nach Anspruch 4, **dadurch gekennzeichnet, dass** die Entfernungsvorrichtung (42) mit der Kamera (10) verbunden ist.

## Claims

1. An endoscopic camera for minimally invasive surgery, comprising
a camera (10) having an optical system (12),
the optical system (12) being attached to a transparent film (26) and the film (26) being removable,
**characterized in that** the film (26) comprises a plurality of sections (30), wherein an upper side of the section (30) attached to an immediately preceding section and a lower side of the section (30) is attached to an immediately succeeding section, wherein the individual sections are folded upon each other in such a manner that they are arranged in a meandering shape.

2. The endoscopic camera of claim 1, **characterized in that** the attachment between the individual sections (30) can be released and that in particular an adhesive layer (34) is arranged between the individual sections (30).

3. The endoscopic camera of one of claims 1 to 2, **characterized in that** at least one film (20, 22, 24, 26) comprises a tab (26, 36, 38).

4. The endoscopic camera of one of claims 1 to 3, **characterized by** a removal device (42) for removing the transparent film (20, 22, 24, 26).

5. The endoscopic camera of claim 4, **characterized in that** the removal device (42) is connected to the camera (10).

## Revendications

1. Caméra endoscopique pour la chirurgie invasive minimale, avec
une caméra (10) comprenant une optique (12),
l'optique (12) étant attachée à un film transparent (26) et le film (26) pouvant être retiré,
**caractérisée en ce que** le film (26) comprend plusieurs parties (30), une face supérieure de la partie (30) étant attachée à une partie directement précédente et une face inférieure étant attachée à une partie directement successive, les différentes parties étant repliées les unes sur les autres de façon que celles-ci soient disposées à la façon d'un méandre.

2. Caméra endoscopique selon la revendication 1, **caractérisée en ce que** l'attachement entre les différentes parties (30) est amovible et que, notamment, une couche adhésive (34) est disposée entre les différentes parties (30).

3. Caméra endoscopique selon une des revendications 1 à 2, **caractérisée en ce qu'**au moins un film (20, 22, 24, 26) comprend une languette (26, 36, 38).

4. Caméra endoscopique selon l'une des revendications 1 à 3, **caractérisée par** un dispositif d'enlèvement (42) pour enlever le film transparent (20, 22, 24, 26).

5. Caméra endoscopique selon la revendication 4, **caractérisée en ce que** le dispositif d'enlèvement (42) est attaché à la caméra (10).
